# EUROPEAN PATENT SPECIFICATION

(11) **EP 4 285 841 B1**
(45) Date of publication and mention of the grant of the patent: **25.02.2026**
(21) Application number: 23174959.9
(22) Date of filing: 23.05.2023
(51) Int. Cl.: A61B 17/04

(54) **SUTURE-ANCHOR INSERTION DEVICE**
VORRICHTUNG ZUM EINFÜHREN EINES NAHTANKERS
DISPOSITIF D'INSERTION D'ANCRE DE SUTURE

(30) Priority: 30.05.2022 US 202263347021 P
(43) Date of publication of application: 06.12.2023
(73) Proprietor: Ossio Ltd., 3079861 Caesarea (IL)
(72) Inventor: ZEEVI, Tal, 3751203 Pardes Hana - Karkur (IL); ASHPIZ, Ido, 4855084 Rosh Haayin (IL); PREISS-BLOOM, Orahn, 3091649 Zichron Yaakov (IL)
(74) Representative: Birdi, Sandeep Singh

(56) References cited:
- US-A1- 2009 234 387
- US-A1- 2015 245 901
- US-A1- 2016 135 801
- US-A1- 2021 338 225
- US-A1- 2022 079 576

## Description

### FIELD OF EMBODIMENTS OF THE INVENTION

The present invention relates to medical apparatus, and specifically to apparatus for securing soft tissue to bone.

### BACKGROUND

Grafts are typically secured to bone via tissue-affixing sutures. Using traditional methods, the tissue-affixing sutures are knotted by the surgeon performing the procedure. More recently, suture anchor devices have been developed for securing soft tissue to bone in a manner that does not require the surgeon to tie suture knots to secure the tissue to the bone. Some such devices include a tip component that defines an eyelet for holding one or more tissue-affixing sutures within a pilot hole. The tissue-affixing suture(s) are secured into the hole by inserting the eyelet into bone and advancing a plug, such as a cannulated screw, over a shaft of the tip component.

US 2016/135801 A1 discloses relevant prior art.

### SUMMARY OF EMBODIMENTS

The invention is defined by appended claim 1.

### BRIEF DESCRIPTION OF THE DRAWINGS

Figs. 1A and 1B are schematic illustrations of a suture-anchor insertion device, in accordance with some applications of the present invention;
Figs. 2A and 2B are schematic illustrations of the suture-anchor insertion device, respectively, without and with tissue-affixing sutures loaded thereon, in accordance with some applications of the present invention; and
Figs. 3A, 3B, 3C, 3D, 3E, 3F, 3G, 3H, and 3I are schematic illustrations of respective steps of a procedure for inserting a suture anchor into a subject's bone using a suture-anchor insertion device, in accordance with some applications of the present invention.

### DETAILED DESCRIPTION OF EMBODIMENTS

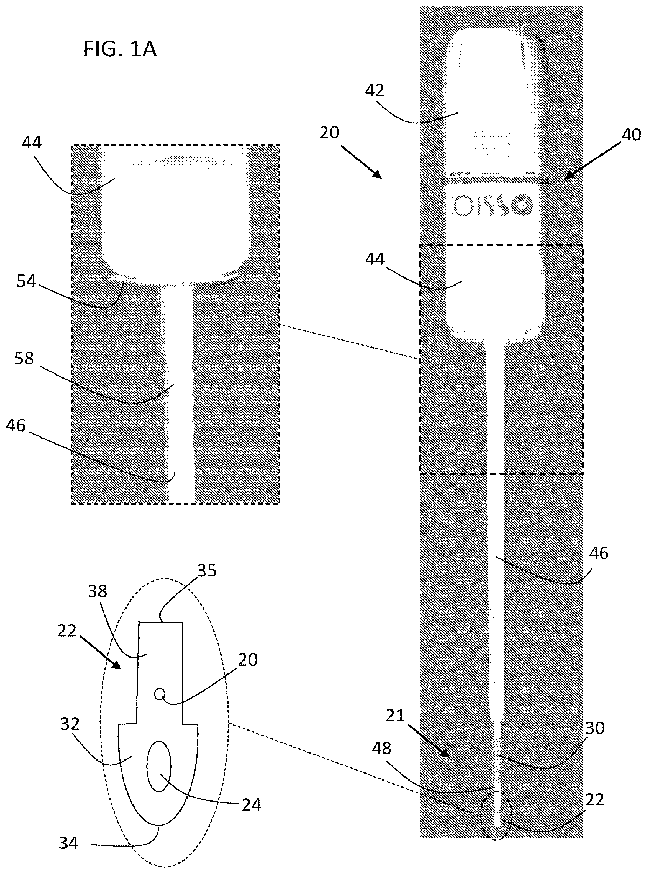

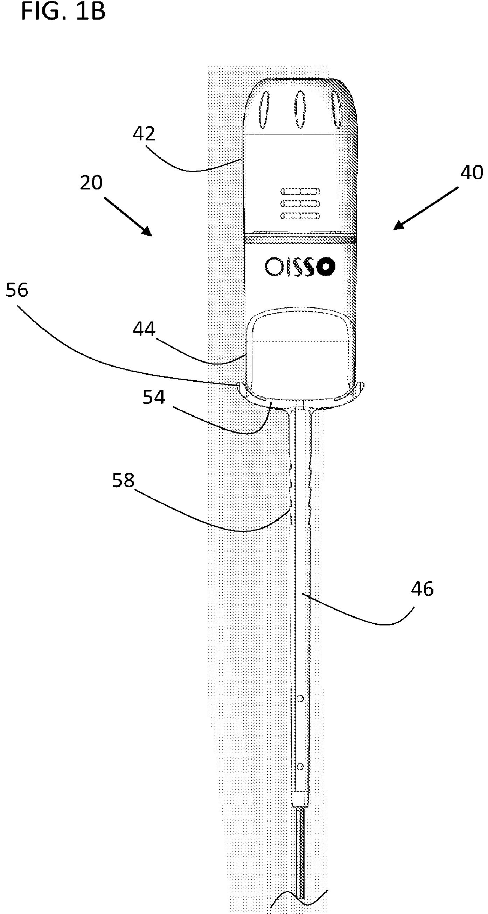

Reference is now made to Figs. 1A and 1B, which are schematic illustrations of a suture-anchor insertion device 20, in accordance with some applications of the present invention. Figs. 1A and 1B show respective examples of the suture-anchor insertion device, with the differences between these examples being described in further detail hereinbelow. In general, all aspects of the suture-anchor insertion devices shown in Figs. 1A and 1B are similar to each other, except for the differences described herein.

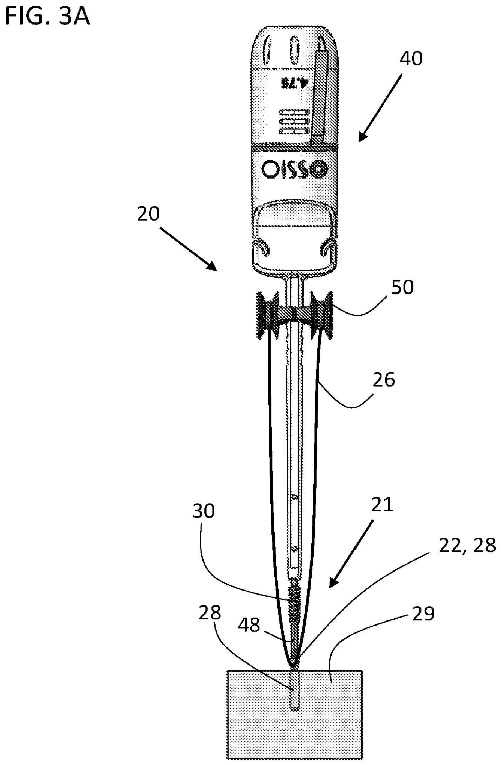

For some applications, suture-anchor insertion device 20 is used to insert a suture anchor 21 into bone, such that the suture anchor secures soft tissue to the bone in a manner that does not require the surgeon to tie suture knots to secure the tissue to the bone. Typically, suture anchor 21 includes a tip component 22 that defines an eyelet 24. The tip component is configured to hold one or more sutures (e.g., between one and four sutures), which are typically tissue-affixing sutures, within a pilot hole 28 in bone 29 (suture 26, pilot hole 28, and bone 29 all shown in Figs. 3A-G). For some applications, some or all of the tissue-affixing sutures are attached to a curved or straight needle at one end or at both ends. The tissue-affixing sutures are typically threaded through one or more tendons or ligaments and are used to approximate the tendons or ligaments to the bone surface and to affix the tendons or ligaments to the bone.

Typically, the eyelet is a closed aperture and the tissue-affixing sutures are suture strands. For some applications (not shown), the eyelet is not a closed aperture. For example, the eyelet may define a portion of a circle, a portion of an ellipse, and/or a forked shape that is configured to hole the suture within the pilot hole. For some applications, tip component 22 defines a widened end portion 32 toward a first (distal) end 34 of the tip component, which is configured to be advanced to the bottom of the pilot hole. The widened end portion typically defines eyelet 24, through which the sutures are threaded. Typically, the tip component includes a shaft 38, which extends axially from the widened end portion of the tip component toward a second (proximal) end 35 of the tip component (which is configured to be placed closer to the top of the pilot hole).

For some applications, the suture anchor includes a plug component, which is configured to secure the tip component within the pilot hole by being advanced into and thereby plugging the pilot hole. Typically, the plug component is a cannulated screw 30, which is configured to secure the tip component within the pilot hole by being screwed into the pilot hole to thereby plug the pilot hole. Cannulated screw 30 typically defines a lumen therethrough, and the cannulated screw is typically advanced over shaft 38 of the tip component, such that the shaft is disposed within the lumen of the cannulated screw.

Suture-anchor insertion device 20 typically includes a handle portion 40 that includes a proximal rotation knob 42 and a distal pusher 44. (The term "proximal" is used herein to denote a portion that is closer to a user of the device (e.g., an orthopedic surgeon), while the term "distal" is used to denote a portion that is farther from the user.) Typically, a pushing rod 46 extends distally from the distal pusher to the proximal end of the cannulated screw. Further typically, a rotation rod 48 extends distally from the proximal rotation knob to the cannulated screw and through the lumen of the cannulated screw. For some applications, the rotation rod extends through the inside of the pushing rod, as shown. For some applications, the cannulated screw is advanced into the pilot hole and over shaft 38 of tip component 22 by using the pushing rod to push the screw distally, while simultaneously using the rotation rod to rotate the screw, and thereby screw the screw into the bone.

Typically, the rotation rod has a cross-sectional shape that conforms with an inner cross-sectional shape the defines the lumen of the cannulated screw, such that the rotation rod engages the internal surfaces of the cannulated screw. For example, the rotation rod may have a polygonal (e.g., a hexagonal shape) and the inner cross-sectional shape that defines the lumen of the cannulated screw may also be polygonal (e.g., hexagonal, as shown). Alternatively, other shapes may be used. Further alternatively or additionally, other methods may be used for rotating the canulated interference screw.

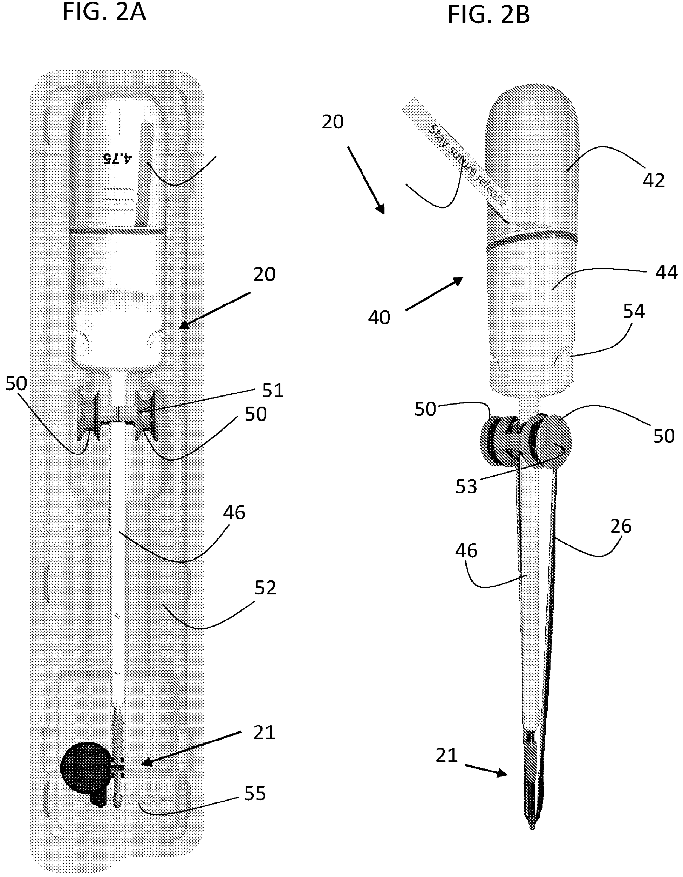

Reference is now made to Fig. 2A, which is a schematic illustrations of suture-anchor insertion device 20 as it is packaged in accordance with some applications of the present invention. Reference is also made to Fig. 2B, which is a schematic illustration of suture-anchor insertion device 20 with proximal ends of tissue-affixing suture(s) 26 being secured by spools 50, in accordance with some applications of the present invention. For some applications, suture-anchor insertion device 20 is provided to a user without the tissue-affixing suture(s) having been preloaded (e.g., as shown in Fig. 2A, in which the suture-anchor insertion device is shown inside packaging 52 in the absence of tissue-affixing suture(s)). Typically, for such applications, the suture-anchor insertion device is provided with a clip 55 that passes through eyelet 24 of tip component 22, in order to facilitate insertion of tissue-affixing suture(s) via the eyelet. Alternatively, the suture-anchor insertion device 20 is provided to a user with tissue-affixing suture(s) having been preloaded. For some such applications, the tissue-affixing suture(s) is provided preloaded through eyelet 24 of tip component 22 and with proximal ends of tissue-affixing suture(s) 26 having been secured by being wound around spools 50, as shown in Fig. 2B. Typically, the proximal ends of the tissue-affixing suture(s) are wound around the spools, such as to prevent damage to the tissue-affixing suture(s) (e.g., twisting and/or knotting of the tissue-affixing suture(s)) while the tissue-affixing suture(s) are disposed within the packaging. Typically, the spools secure the proximal ends of the tissue-affixing suture(s) with respect to pushing rod 46 and/or with respect to handle portion 40.

For some applications, suture-anchor insertion device 20 is provided to a user without the tissue-affixing suture(s) having been preloaded, but the insertion device is provided with spools 50. Typically, once the tissue-affixing suture(s) has been inserted through eyelet 24 of tip component 22 by the user, the user then winds the proximal ends of the tissue-affixing suture(s) around spools 50. Thus, irrespective of whether or not the insertion device is provided with the tissue-affixing suture(s) having been inserted through eyelet 24 of tip component 22, for some applications, prior to insertion of the suture anchor and/or during the insertion of the suture anchor, the proximal ends of the tissue-affixing suture(s) are wound around the spools. Typically, the proximal ends of the tissue-affixing suture(s) are wound around the spools, such as to prevent damage to the tissue-affixing suture(s) (e.g., twisting and/or knotting of the tissue-affixing suture(s)) and to hold the proximal ends of the tissue-affixing suture(s) out of the way of the insertion device. For some applications, prior to insertion of the suture anchor and/or during the insertion of the suture anchor, the user is able to adjust the tension of the tissue-affixing suture(s) by winding the spools. For some applications, suture-anchor insertion device 20 is provided to a user with the tissue-affixing suture(s) having been preloaded onto the spools, and the user removes the proximal ends of the tissue-affixing suture(s) from the spools prior to the insertion of the suture anchor.

As described hereinabove, for some applications, some or all of tissue-affixing sutures 26 are attached to a curved or straight needle at one end or at both ends. For some applications, spools 50 are configured to safely store the needles. For example, the spools may define holes 51 that are shaped so as to receive tips of the needles and to thereby hold the needles in a way that does not expose the sharp tips of the needles. For some applications, the spool is shaped such as to accommodate the needle, for example, the needles may be curved and the radius of curvature of spool is similar to that of the needles, such that the spool accommodates the curvature of the needle.

Typically, after suture anchor 21 has been inserted, tissue-affixing sutures 26 are released from the spools by pulling the tissue-affixing sutures 26 while allowing the spools to rotate and release the strands, for example by holding the sides of the spools lightly and allowing them to rotate. For some applications, the spools define round indents 53 on their outer surfaces such as to allow a user to insert their fingers into the indents and thereby hold the spools while they are rotated as the sutures are pulled off. As noted above, for some applications, the user removes the proximal ends of the tissue-affixing suture(s) from the spools prior to the insertion of the suture anchor.

Typically, spools 50 are detachably attached to the suture-anchor insertion device such that the spools are configured to remain attached while the suture anchor is being inserted but can be readily removed following suture anchor insertion. For some applications, the user removes the spools from the suture-anchor insertion device prior to the insertion of the suture anchor. For some applications, the spools are attached to a rod of the suture-anchor insertion device, e.g., to pushing rod 46 of the suture-anchor insertion device. For some such applications, pushing rod 46 includes a segment 58 having a non-smooth surface (e.g., a segment that defines teeth, waves, and/or a ribbed segment, shown in Fig. 1) in order to facilitate coupling of the spools to the pushing rod. Typically, the suture-anchor insertion device includes two spools (e.g., with one spool on each side of the suture anchor). For some such applications, the spools are coupled to each other. Typically, the spools are removable from the pushing rod with a single motion, for example by pulling the spools from the pushing rod. Typically, the spool is comprised of a material having the same or higher flexibility than the flexibility of the pushing rod.

Referring again to Figs. 1A and 1B (which show respective examples of the suture-anchor insertion device in the absence of suture 26), for some applications, handle portion 40 of insertion device 20 defines shoulder cleats 54. For example, as shown, the distal end of the handle portion (i.e., the distal end of pusher 44) may define shoulder cleats. Typically, the shoulder cleats extend substantially laterally with respect to the handle portion. (It is noted that the shoulder cleats may also extend in additional directions, other than the lateral direction. For example, the shoulder cleats may be shaped to define a proximally-facing concave curvature, as shown.) For some applications, the shoulder cleats extend laterally to the edges of the handle portion, e.g., as shown in Fig. 1A. Alternatively, the shoulder cleats extend laterally past the lateral edges of the handle portion, e.g., as shown in Fig. 1B. For some applications, as an alternative to or in addition to winding the proximal ends of tissue-affixing suture(s) 26 around spools 50, the proximal ends of tissue-affixing suture(s) 26 are inserted into the shoulder cleats. For some such applications, the tissue-affixing suture(s) is provided preloaded through eyelet 24 of tip component 22 and with proximal ends of tissue-affixing suture(s) 26 having been secured (typically, with respect to the handle portion) by having been inserted into the shoulder cleats. Typically, the proximal ends of the tissue-affixing suture(s) are secured by the shoulder cleats, such as to prevent damage to the tissue-affixing suture(s) (e.g., twisting and/or knotting of the tissue-affixing suture(s)) while the tissue-affixing suture(s) are disposed within the packaging. For some applications, suture-anchor insertion device 20 is provided to a user without the tissue-affixing suture(s) having been preloaded, but the insertion device is provided with shoulder cleats 54. Typically, once the tissue-affixing suture(s) has been inserted through eyelet 24 of tip component 22 by the user, the user then inserts the proximal ends of the tissue-affixing suture(s) into shoulder cleats 54. Thus, irrespective of whether or not the insertion device is provided with the tissue-affixing suture(s) having been inserted through eyelet 24 of tip component 22, for some applications, prior to insertion of the suture anchor and/or during the insertion of the suture anchor, the proximal ends of the tissue-affixing suture(s) are inserted into the shoulder cleats. Typically, the proximal ends of the tissue-affixing suture(s) are inserted into the shoulder cleats, such as to prevent damage to the tissue-affixing suture(s) (e.g., twisting and/or knotting of the tissue-affixing suture(s)) and to hold the proximal ends of the tissue-affixing suture(s) out of the way of the insertion device. For some applications, suture-anchor insertion device 20 is provided to a user with the tissue-affixing suture(s) having been inserted into the shoulder cleats, and the user removes the proximal ends of the tissue-affixing suture(s) from the shoulder cleats prior to the insertion of the suture anchor.

Typically, the shoulder cleats include a trapping mechanism (e.g., a male-female interlocking structure) for mechanically trapping the proximal ends of the tissue-affixing suture(s) such that the proximal ends of the tissue-affixing suture(s) cannot be pulled out longitudinally but can be removed by pulling the proximal ends of the tissue-affixing suture(s) laterally. As described hereinabove, for some applications, the shoulder cleats extend laterally outwardly past the lateral edges of the handle portion, e.g., as shown in Fig. 1B. For some applications, suture-anchor insertion device 20 is provided to a user without the tissue-affixing suture(s) having been preloaded, but the insertion device is provided with shoulder cleats 54. Typically, once the tissue-affixing suture(s) has been inserted through eyelet 24 of tip component 22 by the user, the user then inserts the proximal ends of the tissue-affixing suture(s) into shoulder cleats 54. For some such applications, lateral extensions 56 of the shoulder cleats (which extend laterally past the lateral edges of the handle portion), allow the user to initially adjust the tension of the tissue-affixing suture(s) by pulling proximal ends of the tissue-affixing suture(s) around the lateral extensions. Subsequently, the tissue-affixing suture(s) are secured at the desired tightness by sliding the proximal ends of the tissue-affixing suture(s) laterally into the trapping mechanism.

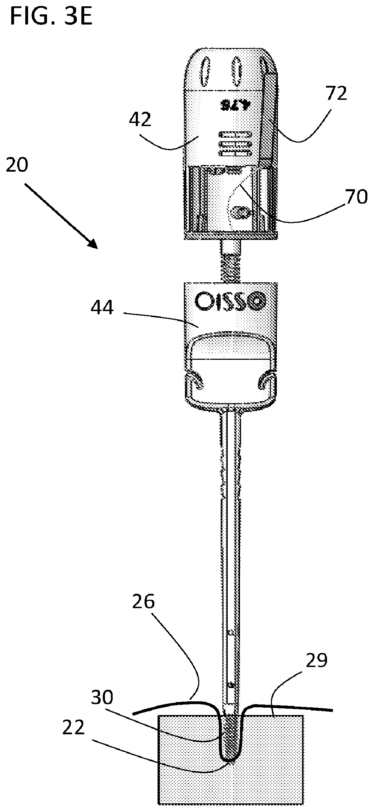

According to the invention, the suture-anchor insertion device includes a stay suture 70 (shown in Figs. 3E-G, for example), and a stay-suture release tab 72. The stay suture typically passes through a stay-suture eyelet 74 defined by shaft 38 of tip component 22 of the suture anchor, and then passes through a lumen defined by rotation rod 48 to handle portion 40 (e.g., to rotation knob 42). The stay suture is typically provided preloaded within the insertion device such that the tip component is fixed to the distal end of the rotation rod. At its proximal end, the stay suture is held in position with respect to the rotation knob via the stay-suture release tab. In order to release the tip component from the insertion device once the suture anchor has been properly implanted, the stay-suture release tab is typically manually removed (e.g., pulled away and/or ripped). This is described in further detail hereinbelow with reference to Figs. 3E-H.

Typically, the suture-anchor insertion device is provided to the user with cannulated screw 30 pre-mounted on rotation rod 48 above tip component 22, such that when the proximal end of the cannulated screw is in contact with the distal end of pushing rod 46, there is an axial gap between the cannulated screw and the tip component. For some applications, the length of the gap is approximately equal to the length of the pilot hole.

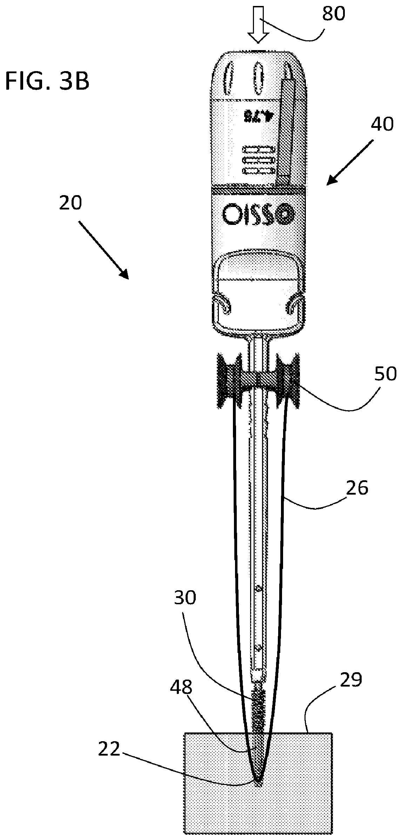

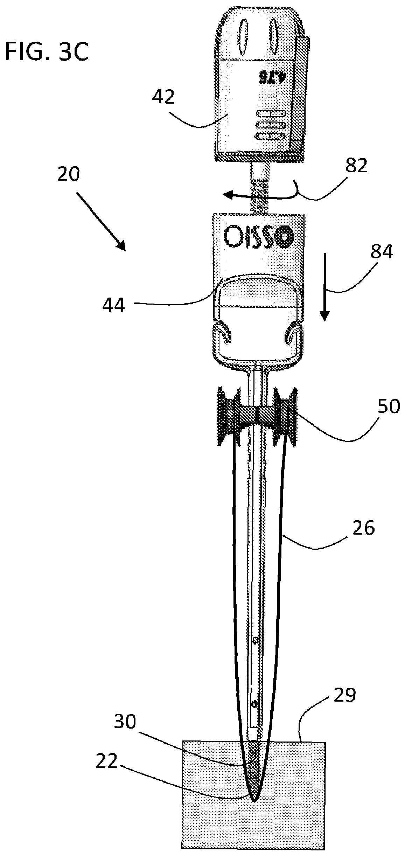

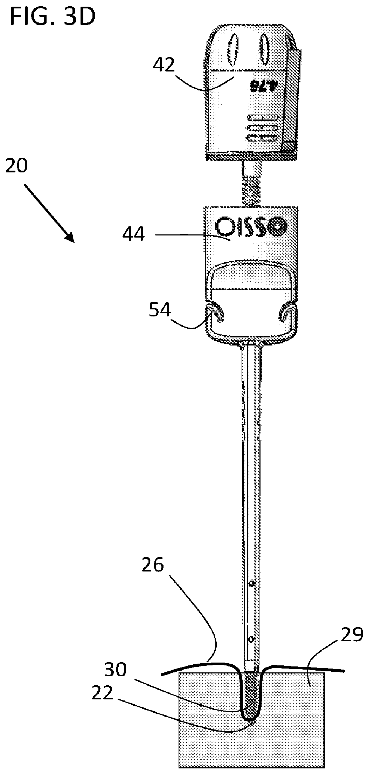

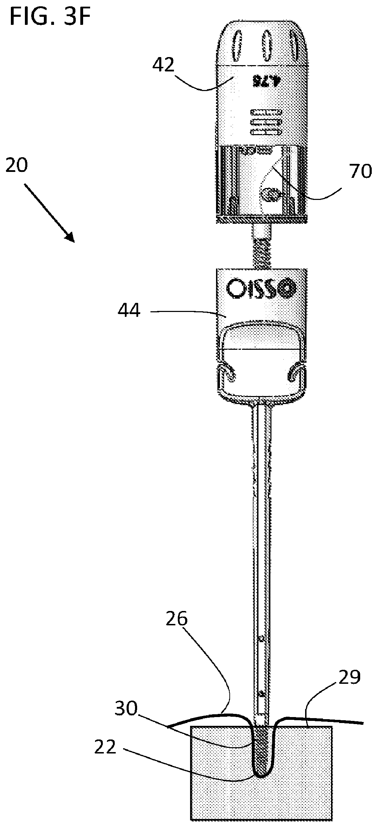

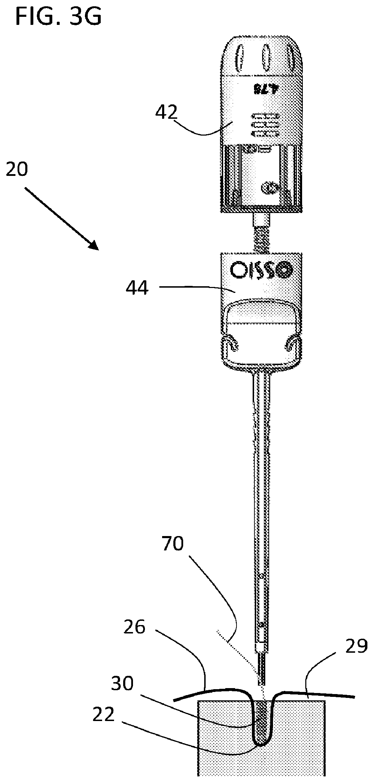

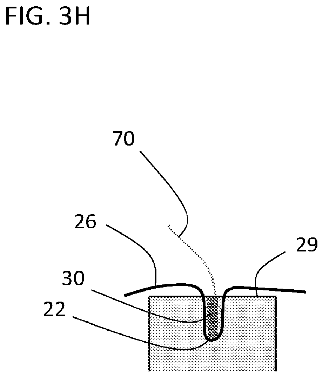

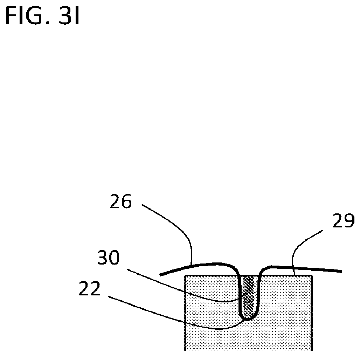

Reference is now made to Figs. 3A, 3B, 3C, 3D, 3E, 3F, 3G, 3H, and 3I, which are schematic illustrations of respective steps of a procedure in which a suture 26 is secured to bone 29, in accordance with some applications of the present invention.

Typically, in a first step, pilot hole 28 is drilled into bone 29. Fig. 3A schematically illustrates bone 29 with pilot hole 28 having already been drilled, and with the suture-anchor insertion device prepared for insertion of the suture anchor into the pilot hole, with the tissue-fixation suture(s) inserted through eyelet 24 of tip component 22 and with the proximal ends of the tissue-fixation suture(s) wound around spools 50. (As described hereinabove, alternatively or additionally, the proximal ends of the tissue-fixation suture(s) wound around shoulder cleats 54.) For some applications, pilot hole 28 is equal to the total length of the suture anchor 21. Alternatively, the pilot hole is shorter than the total length of the suture anchor 21, e.g., up to half of the total length of the suture anchor. Cannulated screw 30 is mounted on rotation rod 48 above tip component 22, with an axial gap between the cannulated screw and the tip component. As described hereinabove, for some applications, the length of the gap is approximately equal to the length of the pilot hole.

Referring now to Fig. 3B, in a subsequent step, tip component 22 and rotation rod 48 are advanced into the pilot hole. For some applications, the tip component 22 and rotation rod 48 are advanced into the pilot hole by gently tapping an upper end of handle portion 40, as indicated by arrow 80. For some applications, the rotation rod includes a marking (e.g., a laser-cut marking) to indicate a depth to which to advance the rotation rod into the pilot hole. As noted above, for some applications, the pilot hole is shorter than the total length of the suture anchor 21, e.g., up to half of the total length of the suture anchor. For such applications, the tip component is advanced into bone 29 to a greater depth than the depth of the pilot hole that was drilled. Typically, this is possible because cancellous bone tissue is spongy. Further typically, this results in the tip component being more securely anchored in place within the pilot hole (and the suture thereby being more securely anchored within the pilot hole) relative to the tip element is advanced into bone 29 only as far as the depth of the pilot hole that was drilled. Typically, the rotation rod 48 is advanced into the pilot hole, such that the distal end of cannulated screw 30 is disposed at the entrance to the pilot hole.

Referring now to Fig. 3C, typically, the cannulated screw is screwed into the pilot hole by simultaneously (a) rotating rotation rod 48 (as indicated by arrow 82), and (b) pushing cannulated screw 30 axially along rotation rod 48 using pushing rod 46 (as indicated by arrow 84).

Referring to Fig. 3D, once the cannulated screw has been fully screwed into the pilot hole (and typically, once the distal end of the screw is fully advanced over shaft 38 of tip component 22, such that the widened end portion of the tip component blocks the screw from advancing further with respect to the tip component), the proximal ends of the tissue-fixation suture(s) are released. Typically, after suture anchor 21 has been inserted, tissue-affixing sutures 26 are released from the spools by pulling the tissue-affixing sutures 26 while allowing the spools to rotate and release the sutures, for example by holding the sides of the spools lightly and allowing them to rotate. As described hereinabove, for some applications, the spools define round indents 53 (shown in Fig. 2B) on their outer surfaces such as to allow a user to insert their fingers into the indents and thereby hold the spools while they are rotated as the sutures are pulled off. As noted above, for some applications, the user removes the proximal ends of the tissue-affixing suture(s) from the spools prior to the insertion of the suture anchor.

As described hereinabove, typically, spools 50 are detachably attached to the suture-anchor insertion device such that the spools are configured to remain attached while the suture anchor is being inserted but can be readily removed following suture anchor insertion. For some applications, the user removes the spools from the suture-anchor insertion device prior to the insertion of the suture anchor. Typically, the suture-anchor insertion device includes two spools, with one on each side of the suture anchor. For some such applications, the spools are coupled to each other. Typically, the spools are removable from the pushing rod with a single motion, for example by pulling the spools from the pushing rod. Typically, the spool is comprised of a material having the same or higher flexibility than the flexibility of the pushing rod.

For applications in which the proximal ends of the tissue-affixing suture(s) are inserted into shoulder cleats 54, the proximal ends of the tissue-affixing suture(s) are typically removed from the shoulder cleats at this stage. As described above, typically, the shoulder cleats include a trapping mechanism (e.g., a male-female interlocking mechanism) for mechanically trapping the tissue-affixing suture(s) such that the tissue-affixing suture(s) cannot be pulled out longitudinally but can be removed by pulling the tissue-affixing suture(s) to the side. For such applications, the tissue-affixing suture(s) are released from the mechanism in order to remove them from the shoulder cleats. For some applications, the user removes the proximal ends of the tissue-affixing suture(s) from the shoulder cleats prior to the insertion of the suture anchor.

Referring to Fig. 3E, as described hereinabove, for some applications, the suture-anchor insertion device includes stay suture 70 and stay-suture release tab 72. The stay suture typically passes through a stay-suture eyelet 74 defined by shaft 38 of tip component 22 of the suture anchor, and then passes through a lumen defined by rotation rod 48 to handle portion 40 (e.g., to rotation knob 42). The stay suture is typically provided preloaded within the insertion device such that the tip component is fixed to the distal end of the rotation rod. At its proximal end, the stay suture is held in position with respect to the rotation knob via the stay-suture release tab (e.g., by the stay-suture release tab adhering the stay suture to an inner surface of the rotation knob). Fig. 3E shows the rotation knob with a portion of its cover removed, such that coupling between the stay suture and the stay-suture release tab is visible.

In order to release the tip component from the insertion device once the suture anchor has been properly implanted, the stay-suture release tab is typically manually removed (e.g. pulled away and/or ripped). For some applications, the stay-suture release tab is made of a manually tearable material, such as paper or fabric, that is configured to be manually torn such as to release the stay suture. Alternatively, the stay-suture release tab is made of is made of a non-tearable material (e.g., a material made of high-density spunbound polyethylene fibers, such as Tvvek^{®}) that is configured to be pulled away such as to release the stay suture. In an alternative embodiment (not shown), a mechanical element such as a button or a lever is used to release the stay suture. Further alternatively, the stay suture may be released by opening up the handle portion (e.g., by opening up rotation knob 42 of the handle portion).

Fig. 3F shows the suture-anchor insertion device with the stay-suture release tab having been removed, such that the stay suture is released. Typically, the release of the stay suture decouples the tip component from the distal end of the rotation rod, thereby allowing the rotation rod to be retracted from inside the cannulated screw, as shown in Figs. 3G and 3H. Typically, the stay suture is then simply pulled out of the stay suture eyelet, leaving only the suture anchor (including tissue-affixing suture(s) 26) in placed in the bone, as shown in Fig. 3I. As described hereinabove, the tissue-affixing suture(s) are typically threaded through one or more tendons or ligaments, with the tissue-affixing suture(s) being used to approximate the tendons or ligaments to the bone surface to the affix tendons or ligaments to the bone.

It is noted that an alternative configuration from that described with reference to Figs. 3E-H is for the stay suture to pass from the eyelet of the tip component all the way through an internal cannula of the suture-anchor insertion device and out of the proximal end of the suture-anchor insertion device. For such applications, the stay suture is typically fixated to the suture-anchor insertion device, for example by winding the suture around a crevice at the back of the suture-anchor insertion device. However, in such applications, releasing the stay suture typically requires unwinding of the stay suture from the device, which is burdensome. Using the configuration that is shown in Figs. 3E-H, the stay suture only passes through the rotation rod, which is substantially shorter than the entire length of the insertion device. Furthermore, the stay suture is protected by the rotation rod and the handle portion, such that no portion of the stay suture is externally exposed until the stay suture is released.

It is noted that although some applications of the present disclosure are described as being applied to tissue-affixing suture(s), the scope of the present disclosure includes applying the apparatus and methods described herein to one or more sutures of any type, *mutatis mutandis.*

## Claims

1. Apparatus for securing a suture to a bone (29), the apparatus comprising:
a suture anchor (21) configured to be inserted into a hole (28) in the bone, the suture anchor comprising:
a) a tip component (22) configured to be advanced into the hole, the tip component comprising:
i) a widened end portion (32) that defines an eyelet (24) that is configured to receive the suture (26);
ii) a shaft (38) that projects axially from the widened end portion;
iii) a stay suture eyelet (74); and
b) a cannulated screw (30) configured to secure the tip component within the hole within the bone by being advanced over the shaft of the tip component; and
a suture-anchor insertion device (20) comprising:
c) a handle portion (40);
d) one or more rods (46, 48) configured to advance the cannulated screw into the hole within the bone;
e) a stay suture (70) configured to pass through the stay suture eyelet and into the handle portion, by passing along a lumen defined by one of the one or more rods;
f) a stay-suture release tab (72) configured to releasably hold a proximal end of the stay suture within the handle portion; and
g) one or more spools (50) configured to releasably secure proximal ends of the suture by the proximal ends of the suture being wound around the one or more spools; or
one or more shoulder cleats (54) extending substantially laterally at a distal end of the handle portion, the shoulder cleats being configured to releasably secure proximal ends of the suture by the proximal ends of the suture being inserted into the one or more shoulder cleats.

2. The apparatus according to claim 1, wherein the one or more spools (50) are configured to secure the proximal ends of the suture (26), to prevent twisting and/or knotting of the suture.

3. The apparatus according to claim 1, wherein the one or more spools (50) are configured to secure the proximal ends of the suture (26), to hold the proximal ends of the suture out of the way of the suture-anchor insertion device (20).

4. The apparatus according to claim 1, wherein the apparatus comprises the suture (26), and wherein the suture anchor insertion device (20) is provided to a user with the suture passing through the eyelet (24) and with proximal ends of the suture wound around the one or more spools (50), or wherein the suture anchor insertion device is configured to be provided to a user without the suture passing through the eyelet and wherein the apparatus is configured for a user to insert the suture through the eyelet and to wind the proximal ends of the suture around the one or more spools.

5. The apparatus according to claim 1, wherein the one or more spools (50) are configured so that the proximal ends of the sutures (26) are configured to be released from the one or more spools prior to advancing the suture anchor (21) into the hole (28) in the bone (29), or wherein the one or more spools are configured so that the proximal ends of the sutures are configured to be released from the one or more spools subsequent to advancing the suture anchor into the hole in the bone, or wherein the one or more spools are configured to allow a user to adjust tension of the suture by winding the one or more spools.

6. The apparatus according to claim 1, wherein the apparatus is configured for use with a suture (26) that is coupled to a needle, and wherein the one or more spools (50) are configured to accommodate the needle, or wherein the apparatus is configured for use with a suture that is coupled to a curved needle, and wherein the one or more spools has a curvature that is such as to accommodate a curvature of the curved needle or, wherein the one or more spools define one or more holes (51) that are configured to receive a tip of the needle.

7. The apparatus according to claim 1, wherein the one or more spools (50) are detachably coupled to the one or more rods (46, 48), or wherein the one or more spools are configured to be detached from the one or more rods prior to advancing the suture anchor (21) into the hole (28) in the bone (29), or wherein the one or more spools are configured to be detached from the one or more rods subsequent to advancing the suture anchor into the hole in the bone.

8. The apparatus according to claim 7, wherein the one or more rods (46, 48) include a segment (58) having a non-smooth surface to facilitate coupling of the one or more spools (50) to the one or more rods

9. The apparatus according to claim 7, wherein the one or more spools (50) comprises two or more spools that are coupled to each other, for detaching from the one or more rods (46, 48) simultaneously with each other, or wherein the two or more spools are configured to be detached from the one or more rods with a single motion, or wherein one or more spools are configured such that the suture (26) is released from the spools by pulling the sutures while allowing the spools to rotate and release the suture, or wherein the one or more spools define round indents (53) on outer surfaces thereof, which are configured to allow a user to insert their fingers into the indents and thereby hold the spools while the spools are rotated.

10. The apparatus according to claim 1, wherein the one or more rods (46, 48) comprise a rotation rod (48) that is configured to rotate the cannulated screw (30), or wherein the one or more rods further comprise a pushing rod (46) configured to push the cannulated screw, while it is being rotated, or wherein the one or more spools (50) are coupled to the pushing rod.

11. The apparatus according to claim 1, wherein the one or more shoulder cleats (54) are configured to secure the proximal ends of the suture (26), to prevent twisting and/or knotting of the suture, or wherein the one or more shoulder cleats are configured to secure the proximal ends of the suture, to hold the proximal ends of the suture out of the way of the suture-anchor insertion device (20).

12. The apparatus according to claim 1, wherein the suture anchor insertion device (20) is configured to be provided to a user without the suture (26) passing through the eyelet (24) and wherein the apparatus is configured for a user to insert the suture through the eyelet and to insert the proximal ends of the suture into the one or more shoulder cleats (54), or wherein the one or more shoulder cleats are configured such that the proximal ends of the sutures are configured to be released from the one or more shoulder cleats prior to advancing the suture anchor (20) into the hole (28) in the bone (29), or wherein the one or more shoulder cleats are configured so that the proximal ends of the sutures are configured to be released from the one or more shoulder cleats subsequent to advancing the suture anchor into the hole in the bone, or wherein the one or more shoulder cleats are configured to allow a user to adjust tension of the suture, or wherein the one or more shoulder cleats comprise a trapping mechanism configured to mechanically trap the proximal ends of the suture so that the proximal ends of the suture cannot be pulled out of the shoulder cleats longitudinally but can be removed from the shoulder cleats by pulling the proximal ends of the suture laterally.

13. The apparatus according to claim 1, wherein the shoulder cleats (54) extend laterally, to define lateral extensions that extend laterally past lateral edges of the handle portion (40), or wherein the lateral extensions of the shoulder cleats are configured to allow a user to adjust tension of the suture (26) by pulling the proximal ends of the suture around the lateral extensions, or wherein the shoulder cleats define a trapping mechanism and are configured to allow the user to secure the suture at a desired tightness by sliding the proximal ends of the suture laterally from the lateral extensions and into the trapping mechanism.

## Patentansprüche

1. Vorrichtung zum Befestigen eines Fadens an einem Knochen (29), wobei die Vorrichtung Folgendes umfasst:
einen Fadenanker (21), der dazu konfiguriert ist, in ein Loch (28) im Knochen eingesetzt zu werden, wobei der Fadenanker umfasst:
a) eine Spitzenkomponente (22), die dazu konfiguriert ist, in das Loch vorgeschoben zu werden, wobei die Spitzenkomponente umfasst:
i) einen aufgeweiteten Endabschnitt (32), der eine Öse (24) definiert, die dazu konfiguriert ist, den Faden (26) aufzunehmen;
ii) einen Schaft (38), der axial von dem aufgeweiteten Endabschnitt vorragt;
iii) eine Haltefadenöse (74); und
b) eine kanülierte Schraube (30), die dazu konfiguriert ist, die Spitzenkomponente innerhalb des Lochs im Knochen zu befestigen, indem sie über den Schaft der Spitzenkomponente vorgeschoben wird; und
eine Fadenanker-Einbringvorrichtung (20), umfassend:
c) einen Griffabschnitt (40);
d) einen oder mehrere Stäbe (46, 48), die dazu konfiguriert sind, die kanülierte Schraube in das Loch innerhalb des Knochens vorzuschieben;
e) einen Haltefaden (70), der dazu konfiguriert ist, durch die Haltefadenöse und in den Griffabschnitt zu verlaufen, indem er entlang eines Lumens verläuft, das durch einen der einen oder mehreren Stäbe definiert wird;
f) eine Haltefaden-Freigabelasche (72), die dazu konfiguriert ist, ein proximales Ende des
Haltefadens lösbar innerhalb des Griffabschnitts zu halten; und
g) eine oder mehrere Spulen (50), die dazu konfiguriert sind, proximale Enden des Fadens lösbar zu sichern, indem die proximalen Enden des Fadens um die eine oder mehreren Spulen gewickelt werden; oder
eine oder mehrere Schulterklampen (54), die sich im Wesentlichen seitlich an einem distalen Ende des Griffabschnitts erstrecken, wobei die Schulterklampen dazu konfiguriert sind, proximale Enden des Fadens lösbar zu sichern, indem die proximalen Enden des Fadens in die eine oder mehreren Schulterklampen eingeführt werden.

2. Vorrichtung nach Anspruch 1, wobei die eine oder mehreren Spulen (50) dazu konfiguriert sind, die proximalen Enden des Fadens (26) zu sichern, um ein Verdrehen und/oder Verknoten des Fadens zu verhindern.

3. Vorrichtung nach Anspruch 1, wobei die eine oder mehreren Spulen (50) dazu konfiguriert sind, die proximalen Enden des Fadens (26) zu sichern, um die proximalen Enden des Fadens aus dem Weg der Fadenanker-Einbringvorrichtung (20) zu halten.

4. Vorrichtung nach Anspruch 1, wobei die Vorrichtung den Faden (26) umfasst, und wobei die Fadenanker-Einbringvorrichtung (20) einem Benutzer bereitgestellt wird, wobei der Faden durch die Öse (24) verläuft und wobei proximale Enden des Fadens um die eine oder mehreren Spulen (50) gewickelt sind, oder wobei die Fadenanker-Einbringvorrichtung dazu konfiguriert ist, einem Benutzer bereitgestellt zu werden, ohne dass der Faden durch die Öse verläuft, und wobei die Vorrichtung so konfiguriert ist, dass ein Benutzer den Faden durch die Öse einführt und die proximalen Enden des Fadens um die eine oder mehreren Spulen wickelt.

5. Vorrichtung nach Anspruch 1, wobei die eine oder mehreren Spulen (50) so konfiguriert sind, dass die proximalen Enden des Fadens (26) dazu konfiguriert sind, von der einen oder den mehreren Spulen gelöst zu werden, bevor der Fadenanker (21) in das Loch (28) im Knochen (29) vorgeschoben wird, oder wobei die eine oder mehreren Spulen so konfiguriert sind, dass die proximalen Enden des Fadens dazu konfiguriert sind, von der einen oder den mehreren Spulen gelöst zu werden, nachdem der Fadenanker in das Loch im Knochen vorgeschoben wurde, oder wobei die eine oder mehreren Spulen dazu konfiguriert sind, einem Benutzer zu ermöglichen, die Spannung des Fadens durch Drehen der einen oder mehreren Spulen anzupassen.

6. Vorrichtung nach Anspruch 1, wobei die Vorrichtung zur Verwendung mit einem Faden (26) konfiguriert ist, der mit einer Nadel gekoppelt ist, und wobei die eine oder mehreren Spulen (50) dazu konfiguriert sind, die Nadel aufzunehmen, oder wobei die Vorrichtung zur Verwendung mit einem Faden konfiguriert ist, der mit einer gebogenen Nadel gekoppelt ist, und wobei die eine oder mehreren Spulen eine Krümmung aufweisen, die so beschaffen ist, dass sie eine Krümmung der gebogenen Nadel aufnimmt, oder wobei die eine oder mehreren Spulen ein oder mehrere Löcher (51) definieren, die dazu konfiguriert sind, eine Spitze der Nadel aufzunehmen.

7. Vorrichtung nach Anspruch 1, wobei die eine oder mehreren Spulen (50) lösbar mit dem einen oder den mehreren Stäben (46, 48) gekoppelt sind, oder wobei die eine oder mehreren Spulen dazu konfiguriert sind, von dem einen oder den mehreren Stäben gelöst zu werden, bevor der Fadenanker (21) in das Loch (28) im Knochen (29) vorgeschoben wird, oder wobei die eine oder mehreren Spulen dazu konfiguriert sind, von dem einen oder den mehreren Stäben gelöst zu werden, nachdem der Fadenanker in das Loch im Knochen vorgeschoben wurde.

8. Vorrichtung nach Anspruch 7, wobei der eine oder die mehreren Stäbe (46, 48) ein Segment (58) mit einer nicht-glatten Oberfläche beinhalten, um das Koppeln der einen oder mehreren Spulen (50) mit dem einen oder den mehreren Stäben zu erleichtern.

9. Vorrichtung nach Anspruch 7, wobei die eine oder mehreren Spulen (50) zwei oder mehr Spulen umfassen, die miteinander gekoppelt sind, um sich gleichzeitig von den einen oder mehreren Stäben (46, 48) zu lösen, oder wobei die zwei oder mehr Spulen dazu ausgebildet sind, sich mit einer einzigen Bewegung von den einen oder mehreren Stäben zu lösen, oder wobei eine oder mehrere Spulen so ausgebildet sind, dass der Faden (26) durch Ziehen an dem Faden von den Spulen gelöst wird, wobei sich die Spulen drehen können und den Faden freigeben, oder wobei die eine oder mehrere Spulen runde Vertiefungen (53) auf deren Außenflächen definieren, die dazu konfiguriert sind, einem Benutzer zu ermöglichen, seine Finger in die Vertiefungen einzuführen und dadurch die Spulen zu halten, während die Spulen gedreht werden.

10. Vorrichtung nach Anspruch 1, wobei der eine oder die mehreren Stäbe (46, 48) einen Rotationsstab (48) umfassen, der dazu konfiguriert ist, die kanülierte Schraube (30) zu drehen, oder wobei der eine oder die mehreren Stäbe ferner einen Schubstab (46) umfassen, der dazu konfiguriert ist, die kanülierte Schraube zu schieben, während sie gedreht wird, oder wobei die eine oder mehreren Spulen (50) mit dem Schubstab gekoppelt sind.

11. Vorrichtung nach Anspruch 1, wobei die eine oder mehreren Schulterklampen (54) dazu konfiguriert sind, die proximalen Enden des Fadens (26) zu sichern, um ein Verdrehen und/oder Verknoten des Fadens zu verhindern, oder wobei die eine oder mehreren Schulterklampen dazu konfiguriert sind, die proximalen Enden des Fadens zu sichern, um die proximalen Enden des Fadens aus dem Weg der Fadenanker-Einbringvorrichtung (20) zu halten.

12. Vorrichtung nach Anspruch 1, wobei die Fadenanker-Einbringvorrichtung (20) dazu konfiguriert ist, einem Benutzer bereitgestellt zu werden, ohne dass der Faden (26) durch die Öse (24) verläuft, und wobei die Vorrichtung so konfiguriert ist, dass ein Benutzer den Faden durch die Öse einführt und die proximalen Enden des Fadens in die eine oder mehreren Schulterklampen (54) einführt, oder wobei die eine oder mehreren Schulterklampen so konfiguriert sind, dass die proximalen Enden der Fäden dazu konfiguriert sind, von der einen oder den mehreren Schulterklampen gelöst zu werden, bevor der Fadenanker (21) in das Loch (28) im Knochen (29) vorgeschoben wird, oder wobei die eine oder mehreren Schulterklampen so konfiguriert sind, dass die proximalen Enden der Fäden dazu konfiguriert sind, von der einen oder den mehreren Schulterklampen gelöst zu werden, nachdem der Fadenanker in das Loch im Knochen vorgeschoben wurde, oder wobei die eine oder mehreren Schulterklampen dazu konfiguriert sind, einem Benutzer zu ermöglichen, die Spannung des Fadens anzupassen, oder wobei die eine oder mehreren Schulterklampen einen Fangmechanismus umfassen, der dazu konfiguriert ist, die proximalen Enden des Fadens mechanisch so einzufangen, dass die proximalen Enden des Fadens nicht in Längsrichtung aus den Schulterklampen gezogen werden können, jedoch durch seitliches Ziehen der proximalen Enden des Fadens aus den Schulterklampen entfernt werden können.

13. Vorrichtung nach Anspruch 1, wobei sich die Schulterklampen (54) seitlich erstrecken, um seitliche Verlängerungen zu definieren, die sich seitlich über die seitlichen Ränder des Griffabschnitts (40) hinaus erstrecken, oder wobei die seitlichen Verlängerungen der Schulterklampen dazu konfiguriert sind, einem Benutzer zu ermöglichen, die Spannung des Fadens (26) anzupassen, indem die proximalen Enden des Fadens um die seitlichen Verlängerungen gezogen werden, oder wobei die Schulterklampen einen Fangmechanismus definieren und dazu konfiguriert sind, dem Benutzer zu ermöglichen, den Faden mit einer gewünschten Spannung zu sichern, durch seitliches Verschieben der proximalen Enden des Fadens von den seitlichen Verlängerungen in den Fangmechanismus.

## Revendications

1. Dispositif permettant de fixer une suture à un os (29) et comprenant :
un ancrage de suture (21) conçu pour être inséré dans un trou (28) de l'os, l'ancrage de suture comprenant :
a) un composant d'extrémité (22) conçu pour être enfoncé dans le trou et comprenant :
i) une partie d'extrémité élargie (32) qui forme un œillet (24) conçu pour recevoir la suture (26) ;
ii) une tige (38) qui fait ressort dans le sens axial à partir de la partie d'extrémité élargie ;
iii) un œillet de suture de maintien (74) ; et
b) une vis canulée (30) conçue pour fixer le composant d'extrémité dans le trou de l'os en l'avançant sur la tige de ce composant ; et
un dispositif d'insertion d'ancrage de suture (20) comprenant :
c) une partie de préhension (40) ;
d) une ou plusieurs tiges (46, 48) conçues pour enfoncer la vis canulée dans le trou à l'intérieur de l'os ;
e) une suture de maintien (70) conçue pour passer à travers l'œillet de suture de maintien et dans la partie de préhension, en passant par une lumière définie par l'une des tiges ;
f) une languette de libération de suture de maintien (72) conçue pour maintenir de manière amovible une extrémité proximale de la suture de maintien dans la partie de préhension ; et
g) une ou plusieurs bobines (50) conçues pour fixer de manière amovible les extrémités proximales de la suture en les enroulant autour de la ou des bobines ; ou
un ou plusieurs taquets d'épaulement (54) s'étendant latéralement à une extrémité distale de la partie de préhension, ces taquets sont conçus pour fixer de manière amovible les extrémités proximales de la suture en insérant les extrémités proximales de la suture dans le ou les taquets d'épaulement.

2. Le dispositif selon la revendication 1, dans lequel une ou plusieurs bobines (50) sont conçues pour fixer les extrémités proximales de la suture (26), afin d'empêcher la suture de se tordre et/ou de faire un nœud.

3. Le dispositif selon la revendication 1, dans lequel une ou plusieurs bobines (50) sont conçues pour fixer les extrémités proximales de la suture (26), afin de maintenir les extrémités proximales de la suture à l'écart du dispositif d'insertion d'ancrage de suture (20).

4. Le dispositif selon la revendication 1, dans lequel il comprend la suture (26), et dans lequel le dispositif d'insertion d'ancrage de suture (20) est fourni à un utilisateur avec la suture passant à travers l'œillet (24) et avec les extrémités proximales de la suture enroulées autour de la ou des bobines (50), ou dans lequel le dispositif d'insertion d'ancrage de suture est configuré de manière à être fourni à un utilisateur sans que la suture passe à travers l'œillet et dans lequel le dispositif est configuré pour qu'un utilisateur insère la suture à travers l'œillet et enroule les extrémités proximales de la suture autour d'une ou plusieurs bobines.

5. Le dispositif selon la revendication 1, dans lequel une ou plusieurs bobines (50) sont conçues de telle sorte que les extrémités proximales des sutures (26) peuvent être libérées de la ou des bobines avant d'enfoncer l'ancrage de suture (21) dans le trou (28) dans l'os (29), ou dans lequel une ou plusieurs bobines sont conçues de telle sorte que les extrémités proximales des sutures peuvent être libérées de la ou des bobines après avoir enfoncé l'ancrage de suture dans le trou de l'os, ou dans lequel une ou plusieurs bobines sont conçues pour permettre à un utilisateur d'ajuster la tension de la suture en enroulant une ou plusieurs bobines.

6. Le dispositif selon la revendication 1, dans lequel le dispositif est conçu pour être utilisé avec une suture (26) reliée à une aiguille, et dans lequel une ou plusieurs bobines (50) sont conçues pour accueillir l'aiguille, ou dans lequel le dispositif est conçu pour être utilisé avec une suture reliée à une aiguille courbée, et dans lequel une ou plusieurs bobines présentent une courbure qui s'adapte à celle de l'aiguille courbée, ou encore où la ou les bobines ont un ou plusieurs trous (51) faits pour recevoir la pointe de l'aiguille.

7. Le dispositif selon la revendication 1, dans lequel une ou plusieurs bobines (50) sont couplées de manière amovible à une ou plusieurs tiges (46, 48), ou dans lequel une ou plusieurs bobines sont conçues pour être détachées de la ou des tiges avant d'enfoncer l'ancrage de suture (21) dans le trou (28) de l'os (29), ou dans lequel une ou plusieurs bobines sont conçues pour être détachées de la ou des tiges après avoir enfoncé l'ancrage de suture dans le trou de l'os.

8. Le dispositif selon la revendication 7, dans lequel une ou plusieurs tiges (46, 48) comprennent un segment (58) ayant une surface non lisse pour faciliter le couplage de la ou des bobines (50) à la ou aux tiges.

9. Le dispositif selon la revendication 7, dans lequel une ou plusieurs bobines (50) comprennent deux ou bobines ou plus, reliées entre elles, pour se détacher simultanément de la ou des tiges (46, 48) en même temps, ou dans lequel deux bobines ou plus sont conçues pour être détachées de la ou des tiges d'un seul coup, ou dans lequel une ou plusieurs bobines sont conçues pour que la suture (26) soit libérée des bobines en tirant sur les sutures tout en laissant les bobines tourner et libérer la suture, ou dans lequel la ou les bobines présentent des indentations rondes (53) sur leurs surfaces extérieures, conçues pour permettre à un utilisateur d'y insérer ses doigts et ainsi tenir les bobines pendant que celles-ci tournent.

10. Le dispositif selon la revendication 1, dans lequel la ou les tiges (46, 48) comprennent une tige de rotation (48) qui est conçue pour faire tourner la vis canulée (30), ou dans lequel la ou les tiges comprennent en outre une tige de poussée (46) conçue pour pousser la vis canulée pendant qu'elle est, ou dans lequel une ou plusieurs bobines (50) sont reliées à la tige de poussée.

11. Le dispositif selon la revendication 1, dans lequel un ou plusieurs taquets d'épaulement (54) sont conçus pour fixer les extrémités proximales de la suture (26), afin d'empêcher celle-ci de se tordre et/ou de faire un noeud, ou dans lequel un ou plusieurs d'épaulement sont conçus pour fixer les extrémités proximales de la suture, afin de maintenir ces extrémités à l'écart du dispositif d'insertion d'ancrage de suture (20).

12. Le dispositif selon la revendication 1, dans lequel le dispositif d'insertion d'ancrage de suture (20) est conçu pour être fourni à un utilisateur sans que la suture (26) passe à travers l'œillet (24) et dans lequel le dispositif est conçu pour qu'un utilisateur insère la suture à travers l'œillet et les extrémités proximales de la suture dans un ou plusieurs taquets d'épaulement (54), ou dans lequel un ou plusieurs taquets d'épaulement sont conçus de telle sorte que les extrémités proximales des sutures sont faites pour être libérées du ou des taquets d'épaulement avant d'enfoncer l'ancrage de suture (20) dans le trou (28) de l'os (29), ou dans lequel le ou les taquets d'épaulement sont faits pour que les extrémités proximales des sutures puissent être libérées du ou des taquets d'épaulement après avoir enfoncé l'ancrage de suture dans le trou de l'os, ou dans lequel un ou plusieurs taquets d'épaulement sont conçus pour permettre à un utilisateur d'ajuster la tension de la suture, ou dans lequel un ou plusieurs taquets d'épaulement comprennent un mécanisme de blocage conçu pour bloquer mécaniquement les extrémités proximales de la suture de manière à ce que les extrémités proximales de la suture ne puissent pas être retirées des taquets d'épaulement dans le sens longitudinal, mais puissent être retirées des taquets d'épaulement en tirant les extrémités proximales de la suture dans le sens latéral.

13. Le dispositif selon la revendication 1, dans lequel les taquets d'épaulement (54) s'étendent sur les côtés, pour créer des extensions latérales allant au-delà des bords latéraux de la partie de préhension (40), ou dans lequel les extensions latérales des taquets d'épaule sont conçues de façon à permettre à un utilisateur d'ajuster la tension de la suture (26) en tirant les extrémités proximales de la suture autour des extensions latérales, ou dans lequel les taquets d'épaulement définissent un mécanisme de blocage et sont conçus pour permettre à l'utilisateur de fixer la suture à une tension souhaitée en faisant glisser latéralement les extrémités proximales de la suture depuis les extensions latérales et dans le mécanisme de blocage.
